# EUROPEAN PATENT SPECIFICATION

(11) **EP 4 311 477 B1**
(45) Date of publication and mention of the grant of the patent: **05.03.2025**
(21) Application number: 22187372.2
(22) Date of filing: 28.07.2022
(51) Int. Cl.: A61B 5/00

(54) **APPARATUS FOR NON-INVASIVE IMAGE-OBSERVING DENSITY OF INTRA-EPIDERMAL NERVE FIBER OF HUMAN SKIN**
GERÄT ZUR NICHT-INVASIVEN BILDAUFZEICHNUNG DER DICHTE DER INTRA-EPIDERMALEN NERVENFASERN DER MENSCHLICHEN HAUT
APPAREIL D'OBSERVATION D'IMAGERIE NON INVASIVE DE LA DENSITÉ DES FIBRES NERVEUSES INTRA-ÉPIDERMIQUES DE LA PEAU HUMAINE

(43) Date of publication of application: 31.01.2024
(73) Proprietor: National Taiwan University, Taipei, 10617 (TW)
(72) Inventor: SUN, CHI-KUANG, 106319 Taipei City (TW); WU, PEI-JHE, 106319 Taipei City (TW); TSENG, HSIAO-CHIEH, 104 Taipei City (TW)
(74) Representative: Ter Meer Steinmeister & Partner

(56) References cited:
- US-A1- 2015 157 254
- LAURIA G. ET AL: "EFNS guidelines on the use of skin biopsy in the diagnosis of peripheral neuropathy", EUROPEAN JOURNAL OF NEUROLOGY, vol. 12, no. 10, 1 October 2005 (2005-10-01), GB, pages 747 - 758, XP093011655, ISSN: 1351-5101, DOI: 10.1111/j.1468-1331.2005.01260.x
- SZU-YU CHEN ET AL: "In Vivo Virtual Biopsy of Human Skin by Using Noninvasive Higher Harmonic Generation Microscopy", IEEE JOURNAL OF SELECTED TOPICS IN QUANTUM ELECTRONICS, IEEE, USA, vol. 16, no. 3, 1 May 2010 (2010-05-01), pages 478 - 492, XP011297371, ISSN: 1077-260X
- PEREIRA MANUEL P. ET AL: "Intraepidermal Nerve Fiber Density: Diagnostic and Therapeutic Relevance in the Management of Chronic Pruritus: a Review", DERMATOLOGY AND THERAPY, vol. 6, no. 4, 11 October 2016 (2016-10-11), pages 509 - 517, XP093011386, ISSN: 2193-8210, [retrieved on 20230104], DOI: 10.1007/s13555-016-0146-1

## Description

### FIELD OF THE INVENTION

The present invention relates to an apparatus for non-invasive image-observing the density of an intra-epidermal nerve fiber in human skin, in which the apparatus includes providing a nonlinear optical microscopy device for capturing an intra-epidermal nerve fiber structural image of an image-capturing area of a to-be tested human skin to observe continuous signals of the intra-epidermal nerve fiber structural image, and calculating the density (or density value) of the intra-epidermal nerve fiber of the to-be-tested human skin through an image processing member, evaluating the level of damages to the intra-epidermal nerve fiber of the to-be-tested human body, and determining whether the human body suffers from related neuropathy, such as peripheral neuropathy.

### BACKGROUND OF THE INVENTION

At present, the approach adopted by hospital neurology departments to observe nerves is to remove skin tissues from a patient by using a punch biopsy. It is an invasive method that not only causes discomfort to human body, but occasionally causes accidental injuries. In addition, when skin tissue images are observed for diagnosis, it requires special chemical immunostaining techniques to complete the observation. The medical procedure is complicated, and its accuracy is yet to be improved.
Lauria et al. (Eur J Neurol. 2005 Oct;12(10):747-58) relates to the EFNS (European Federation of Neurological Societies) guidelines on the use of skin biopsy in the diagnosis of peripheral neuropathy.
Pereira et al. (Dermatol Ther (Heidelb). 2016 Dec;6(4):509-517) is a review article relating to measurement of intraepidermal nerve fiber density by punch biopsy and the diagnostic and therapeutic relevance of intraepidermal nerve fiber density in the management of chronic pruritus.
US 2015/157254 A1 discloses a method for diagnosing skin disease based upon in vivo skin imaging including a harmonic generation microscopy device.

Accordingly, a non-invasive and harmless method for observing signals of an intra-epidermal nerve fiber structural image to obtain the density of the intra-epidermal nerve fiber in the epidermis of the human skin without requiring special immunohistochemistry staining technology to provide rapid and accurate diagnoses and necessary correction is expected by the medical field. Furthermore, it is a breakthrough if the density of the peripheral intra-epidermal nerve fiber can be calculated through observing the peripheral intra-epidermal nerve fiber in the epidermis of human skin to determine if the human is suffering from peripheral neuropathy.

### SUMMARY OF THE INVENTION

The present invention aims to provide an apparatus for non-invasive image-observing the density of an intra-epidermal nerve fiber of human skin, by using the non-invasive harmonic generation microscopy, images of the density of the intra-epidermal nerve fiber of human skin can be observed to evaluate the level of damages to the intra-epidermal nerve fiber of the to-be-tested human body and to determine whether the human body suffers from related neuropathy, such as peripheral neuropathy, in which the peripheral intra-epidermal nerve fiber is an unmyelinated fiber in the epidermis of the human skin.

Another object of the present invention is to provide an apparatus for non-invasive image-observing the density (density value) of the entire segment or one segment of an intra-epidermal nerve fiber in the epidermis of the human skin, which require no process of using special chemical immunostaining technology to obtain the density of the intra-epidermal nerve fiber in human skin, thereby quickly and accurately determining whether the human body suffers from peripheral neuropathy.

The object is solved by the features of the independent claim. Preferred embodiments are given in the dependent claims.

### SUMMARY OF THE DISCLOSURE

A method for non-invasive image-observing the density of an intra-epidermal nerve fiber of human skin is proposed, which comprises:
providing a nonlinear optical microscopy device for capturing an intra-epidermal nerve fiber structural image of an acquisition area of a to-be-tested human skin to observe continuous signals of the intra-epidermal nerve fiber structural image, wherein the continuous signals of the intra-epidermal nerve fiber structural image include a string-like or a claw-like structural signal of a peripheral intra-epidermal nerve fiber formed by crossing through a junctional layer of the human skin epidermis and dermis and extending into the epidermis, and a string-like or a claw-like structural signal of the peripheral intra-epidermal nerve fiber only located in the epidermis, wherein the peripheral intra-epidermal nerve fiber is an unmyelinated fiber in the epidermis of the human skin, and wherein the nonlinear optical microscopy device includes: a laser source for emitting laser light with a pulsed laser, and an image processing member for processing image signals;
focusing the laser light on the intra-epidermal nerve fiber of the acquisition area of the to-be-tested human skin by using the image processing member to obtain continuous signals of the intra-epidermal nerve fiber structural image with a length having at least three points and continuous, wherein the continuous signals of the intra-epidermal nerve fiber structural image are third harmonic generation nonlinear optical signals generated after being excited by the laser light, the size of the signal point of each of the continuous signals of the intra-epidermal nerve fiber structural image is 200 nm or more, and the linear distance from one signal point to another signal point in a three-dimensional space ranges from 0 to 7.5 µm, and wherein the continuous signals connected together by at least three points constitute a string-like intra-epidermal nerve fiber structural signal segment, and the string-like intra-epidermal nerve fiber structural signal segment includes signal segments only existing in the epidermis, and the other signal segments extendedly crossing through the junctional layer of the skin epidermis and dermis to exist in the epidermis, and the shortest distance between the string-like intra-epidermal nerve fiber structural signal segments and the junctional layer of the epidermis and dermis is less than 20µm, thereby constituting a plurality of nerve fibers;
calculating a total number of the plurality of nerve fibers of the total intra-epidermal nerve fiber in the acquisition area of the to-be-tested human skin and dividing it by a total acquisition area (mm²) of the captured images to obtain a density of the intra-epidermal nerve fiber of the to-be-tested human skin; and
evaluating levels of damage to the intra-epidermal nerve fiber of the to-be-tested human skin, and determining whether related neuropathy such as peripheral neuropathy is suffered, that is, the more severe the intra-epidermal nerve fiber is damaged, the lower the density is.

In one or more embodiments, a normal value of the density of the intra-epidermal nerve fiber may be from 50 to 60 (the number of the nerve fibers/mm²).

In one or more embodiments, the intraepidermal nerve fiber may be a C nerve fiber or A-delta nerve fiber, having a point-like structure.

In one or more embodiments, the non-linear optical microscopy device may be a third harmonic generation microscopy device (THG), a second harmonic generation microscopy device (SHG) plus a third harmonic generation microscopy device (THG), or a combination thereof, wherein the specification of the third harmonic generation microscopy device (THG) is NA≧0.75, the central wavelength: 1065-1450nm, pulse width: < 10ps, and reverse collection of optical signals.

In one or more embodiments, each nerve fiber may extend along the same direction or a plurality of directions.

In one or more embodiments, each nerve fiber may be continuous in the same epidermis with no extension, or is continuous in different skin layers with extensions.

In one or more embodiments, when each nerve fiber extends in different skin layers, it may be a point-like signal and a point-like signal extension, or a point-like signal and a linear signal extension.

In one or more embodiments, each nerve fiber may not be arranged as a circle to eliminate cell signals in the skin.

In one or more embodiments, the nonlinear optical microscopy device may be a second harmonic generation microscopy device (SHG) plus a third harmonic generation microscopy device (THG) for observing the intra-epidermal nerve fiber structural image, and if the intra-epidermal nerve fiber extends in the horizontal direction (perpendicular to the forward direction of the laser light), as is known through numerical simulation, it may be is used for generating a high-strength third harmonic generation, so that the intra-epidermal nerve fiber signal is observed in the same skin layer as linear and continuous; and if the intra-epidermal nerve fiber extends in the vertical direction (parallel to the forward direction of the laser light), it may be known through numerical simulation, that if it is a simple cylindrical thin line, the intensity of the third harmonic generation is extremely small, it must conform to the structure of the varicosity to generate high-strength third harmonic generation, so that the intra-epidermal nerve fiber signal is observed in different skin layers as a point-like and point-like extension, or a point-like and linear extension.

In one or more embodiments, the method for non-invasive image-observing the density of an intra-epidermal nerve fiber of human skin may further comprise a displaying step for displaying the image and/or the density value of each nerve fiber.

In one or more embodiments, when observing the intra-epidermal nerve fiber image, the number of cells in a basal layer of the epidermis capable of reflecting visibility of the image may be further observed.

In one or more embodiments, the number of cells in the basal layer may be used to correct the density of the entire segment of the intra-epidermal nerve fiber and the density of the intra-epidermal nerve fiber.

An apparatus for non-invasive image-observing the density of an intra-epidermal nerve fiber of human skin is submitted, which comprises:
a nonlinear optical microscopy device for capturing an intra-epidermal nerve fiber structural image of an acquisition area of a to-be-tested human skin to observe continuous signals of the intra-epidermal nerve fiber structural image, wherein the continuous signals of the intra-epidermal nerve fiber structural image include a string-like or a claw-like structural signal of a peripheral intra-epidermal nerve fiber formed by crossing through a junctional layer of the human skin epidermis and dermis and extending into the epidermis, and a string-like or a claw-like structural signal of the peripheral intra-epidermal nerve fiber only located in the epidermis, wherein the peripheral intra-epidermal nerve fiber is an unmyelinated fiber in the epidermis of the human skin, and wherein the nonlinear optical microscopy device includes: a laser source for emitting laser light with a pulsed laser, and an image processing member for processing image signals, in which the laser light is focused on the intra-epidermal nerve fiber of the acquisition area of the to-be-tested human skin by using the image processing member to obtain continuous signals of the intra-epidermal nerve fiber structural image with a length having at least three points and continuous, wherein the continuous signals of the intra-epidermal nerve fiber structural image are third harmonic generation nonlinear optical signals generated after being excited by the laser light, the size of the signal point of each of the continuous signals of the intra-epidermal nerve fiber structural image is 200 nm or more, and the linear distance from one signal point to another signal point in a three-dimensional space ranges from 0 to 7.5 µm, and wherein the continuous signals connected together by at least three points constitute a string-like intra-epidermal nerve fiber structural signal segment, and the string-like intra-epidermal nerve fiber structural signal segment includes signal segments only existing in the epidermis, and the other signal segments extendedly crossing through the junctional layer of the skin epidermis and dermis to exist in the epidermis, and the shortest distance between the string-like intra-epidermal nerve fiber structural signal segments and the junctional layer of the epidermis and dermis is less than 20µm, thereby constituting a plurality of nerve fibers;
a calculation element for calculating a total number of the plurality of nerve fibers of the total intra-epidermal nerve fiber in the acquisition area of the to-be-tested human skin and dividing it by a total acquisition area (mm²) of the captured images to obtain a density of the intra-epidermal nerve fiber of the to-be-tested human skin; and
an evaluation and judging element for evaluating levels of damage to the intra-epidermal nerve fiber of the to-be-tested human skin, and determining whether related neuropathy such as peripheral neuropathy is suffered, that is, the more severe the intra-epidermal nerve fiber is damaged, the lower the density is.

In one or more embodiments, the apparatus may further comprise a displaying element for displaying the image and/or the density of each nerve fiber.

In one or more embodiments, a normal value of the density of the intra-epidermal nerve fiber may be from 50 to 60 (the number of the nerve fibers/mm²).

In one or more embodiments, the intraepidermal nerve fiber may be a C nerve fiber or A-delta nerve fiber, having a point-like structure.

In one or more embodiments, the non-linear optical microscopy device may be a third harmonic generation microscopy device (THG), a second harmonic generation microscopy device (SHG) plus a third harmonic generation microscopy device (THG), or a combination thereof, wherein the specification of the third harmonic generation microscopy device (THG) is NA≧0.75, the central wavelength: 1065-1450nm, pulse width: < 10ps, and reverse collection of optical signals.

In one or more embodiments, each nerve fiber may extend along the same direction or a plurality of directions.

In one or more embodiments, each nerve fiber may be continuous in the same epidermis with no extension, or is continuous in different skin layers with extensions.

In one or more embodiments, when each nerve fiber extends in different skin layers, it may be a point-like signal and a point-like signal extension, or a point-like signal and a linear signal extension.

In one or more embodiments, each nerve fiber is not arranged as a circle to eliminate cell signals in the skin.

In one or more embodiments, the nonlinear optical microscopy device may be a second harmonic generation microscopy device (SHG) plus a third harmonic generation microscopy device (THG) for observing the intra-epidermal nerve fiber structural image, and if the intra-epidermal nerve fiber extends in the horizontal direction (perpendicular to the forward direction of the laser light), as is known through numerical simulation, it may be used for generating a high-strength third harmonic generation, so that the intra-epidermal nerve fiber signal may be observed in the same skin layer as linear and continuous; and if the intra-epidermal nerve fiber extends in the vertical direction (parallel to the forward direction of the laser light), it may be known through numerical simulation, that if it is a simple cylindrical thin line, the intensity of the third harmonic generation may be extremely small, it must conform to the structure of the varicosity to generate high-strength third harmonic generation, so that the intra-epidermal nerve fiber signal may be observed in different skin layers as a point-like and point-like extension, or a point-like and linear extension.

In one or more embodiments, when observing the intra-epidermal nerve fiber image, the number of cells in a basal layer of the epidermis capable of reflecting visibility of the image may be further observed.

In one or more embodiments, a correction element may be further provided for correcting the density of the intra-epidermal nerve fiber, via using the number of cells in the basal layer.

### BRIEF DESCRIPTION OF THE DRAWINGS

Figure 1 shows a flow chart of a method for non-invasive image-observing the density of an intra-epidermal nerve fiber of human skin.
Figure 2 shows a block diagram of an apparatus for non-invasive image-observing the density of an intra-epidermal nerve fiber of human skin according to the present invention.

### DETAILED DESCRIPTION

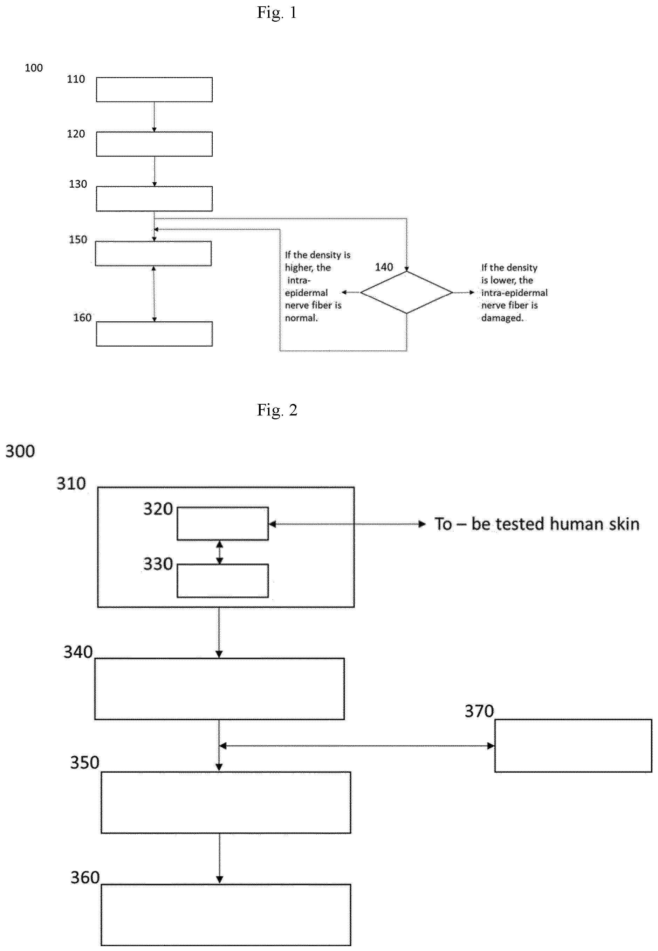

As shown in Figure 1, a method for non-invasive image-observing the density of an intra-epidermal nerve fiber of human skin 100 comprises
providing a nonlinear optical microscopy device for capturing an intra-epidermal nerve fiber structural image of an acquisition area of a to-be-tested human skin to observe continuous signals of the intra-epidermal nerve fiber structural image 110, wherein the continuous signals of the intra-epidermal nerve fiber structural image include a string-like or a claw-like structural signal of a peripheral intra-epidermal nerve fiber formed by crossing through a junctional layer of the human skin epidermis and dermis and extending into the epidermis, and a string-like or a claw-like structural signal of the peripheral intra-epidermal nerve fiber only located in the epidermis, wherein the peripheral intra-epidermal nerve fiber is an unmyelinated fiber in the epidermis of the human skin, and wherein the nonlinear optical microscopy device includes: a laser source for emitting laser light with a pulsed laser, and an image processing member for processing image signals;
focusing the laser light on the intra-epidermal nerve fiber of the acquisition area of the to-be-tested human skin by using the image processing member to obtain continuous signals of the intra-epidermal nerve fiber structural image with a length having at least three points and continuous 120, wherein the continuous signals of the intra-epidermal nerve fiber structural image are third harmonic generation nonlinear optical signals generated after being excited by the laser light, the size of the signal point of each of the continuous signals of the intra-epidermal nerve fiber structural image is 200 nm or more, and the linear distance from one signal point to another signal point in a three-dimensional space ranges from 0 to 7.5 µm, and wherein the continuous signals connected together by at least three points constitute a string-like intra-epidermal nerve fiber structural signal segment, and the string-like intra-epidermal nerve fiber structural signal segment includes signal segments only existing in the epidermis, and the other signal segments extendedly crossing through the junctional layer of the skin epidermis and dermis to exist in the epidermis, and the shortest distance between the string-like intra-epidermal nerve fiber structural signal segments and the junctional layer of the epidermis and dermis is less than 20µm, thereby constituting a plurality of nerve fibers;
calculating a total number of the plurality of nerve fibers of the total intra-epidermal nerve fiber in the acquisition area of the to-be-tested human skin and dividing it by a total acquisition area (mm²) of the captured images to obtain a density of the intra-epidermal nerve fiber of the to-be-tested human skin 130; and
evaluating levels of damage to the intra-epidermal nerve fiber of the to-be-tested human skin, and determining whether related neuropathy such as peripheral neuropathy is suffered, that is, the more severe the intra-epidermal nerve fiber is damaged, the lower the density is 140.

In the method for non-invasive image-observing the density of an intra-epidermal nerve fiber of human skin, a normal value of the density of the intra-epidermal nerve fiber is from 50 to 60 (the number of the nerve fibers/mm²), and wherein the intra-epidermal nerve fiber is a C nerve fiber or A-delta nerve fiber, having a point-like structure.

In the method for non-invasive image-observing the density of an intra-epidermal nerve fiber of human skin 100, the non-linear optical microscopy device is a third harmonic generation microscopy device (THG), a second harmonic generation microscopy device (SHG) plus a third harmonic generation microscopy device (THG), or a combination thereof, wherein the specification of the third harmonic generation microscopy device (THG) is NA≧0.75, the central wavelength: 1065-1450nm, pulse width: < 10ps, and reverse collection of optical signals.

According to the method for non-invasive image-observing the density of an intra-epidermal nerve fiber of human skin 100, wherein each nerve fiber extends along the same direction or a plurality of directions; or each nerve fiber is continuous in the same epidermis with no extension or is continuous in different skin layers with extensions; or each nerve fiber extends in different skin layers, which is a point-like signal and a point-like signal extension, or a point-like signal and a linear signal extension; or each nerve fiber is not arranged as a circle to eliminate cell signals in the skin.

In the method for non-invasive image-observing the density of an intra-epidermal nerve fiber of human skin 100, the nonlinear optical microscopy device is a second harmonic generation microscopy device (SHG) plus a third harmonic generation microscopy device (THG) for observing the intra-epidermal nerve fiber structural image, and if the intra-epidermal nerve fiber extends in the horizontal direction (perpendicular to the forward direction of the laser light), and as is known through numerical simulation, it is used for generating a high-strength third harmonic generation, so that the intra-epidermal nerve fiber signal is observed in the same skin layer as linear and continuous;
and if the intra-epidermal nerve fiber extends in the vertical direction (parallel to the forward direction of the laser light), it is known through numerical simulation, that if it is a simple cylindrical thin line, the intensity of the third harmonic generation is extremely small, it must conform to the structure of the varicosity to generate high-strength third harmonic generation, so that the intra-epidermal nerve fiber signal is observed in different skin layers as a point-like and point-like extension, or a point-like and linear extension.

Further, the method for non-invasive image-observing the density of an intra-epidermal nerve fiber of human skin 100 further comprises a displaying step for displaying the image and/or the density value of each nerve fiber 150.

Still further, method for non-invasive image-observing the density of an intra-epidermal nerve fiber of human skin 100, a correction step 160 is provided as follow. Wherein when observing the intra-epidermal nerve fiber image, the number of cells in a basal layer of the epidermis capable of reflecting visibility of the image is further observed, wherein the number of cells in the basal layer is used to correct the density of the entire segment of the intra-epidermal nerve fiber and the density of the intra-epidermal nerve fiber.

According to the method, examples of the observed and calculated density are given below.

### Example 1:

For a single piece of picture (image) at a size of 0.5×0.5m², taken by the non-invasive optical microscopy device of the invention, it requires a total size of at least greater than 1 mm² for capturing so as to calculate the density of the intra-epidermal nerve fiber in the skin and thus it needs at least four pictures.

Assuming 1mm² of the total size is taken with pictures Nos. 1, 2, 3 and 4:
Picture 1, the calculated number of the nerve fiber is 12;
Picture 2, the calculated number of the nerve fiber is 15;
Picture 3, the calculated number of the nerve fiber is 18; and
Picture 4, the calculated number of the nerve fiber is 10.

The density of the intra-epidermal nerve fibers is 12+15+18+55=55 fibers/mm².

### Example 2:

Assuming a total size of greater than 1mm² is taken with pictures 1, 2, 3, 4, 5 and 6:
Picture 1, the calculated number of the nerve fiber is 12;
Picture 2, the calculated number of the nerve fiber is 15;
Picture 3, the calculated number of the nerve fiber is 18; and
Picture 4, the calculated number of the nerve fiber is 10.
Picture 5, the calculated number of the nerve fiber is 10.
Picture 6, the calculated number of the nerve fiber is 11.

The total number of the nerve fibers is 12+15+18+10+10+11=76 fibers, which are divided by the total size of 0.5×0.5×6=1.5mm².

Then, the density of the intra-epidermal nerve fiber of 76/1.5=50.66 fibers/mm² is obtained.

Via a test of the density of a to-be-tested people suffering the peripherals neuropathy, the density is 26.35 fibers/mm², while the density of a normal people under test is 55.26 fibers/mm². Therefore, the lower the density of the intra-epidermal nerve fiber is, the severe the intra-epidermal is damaged.

As shown in Figure 2, an apparatus for non-invasive image-observing the density of an intra-epidermal nerve fiber of human skin 300 according to the invention comprises:
a nonlinear optical microscopy device 310 for capturing an intra-epidermal nerve fiber structural image of an acquisition area of a to-be-tested human skin to observe continuous signals of the intra-epidermal nerve fiber structural image, wherein the continuous signals of the intra-epidermal nerve fiber structural image include a string-like or a claw-like structural signal of a peripheral intra-epidermal nerve fiber formed by crossing through a junctional layer of the human skin epidermis and dermis and extending into the epidermis, and a string-like or a claw-like structural signal of the peripheral intra-epidermal nerve fiber only located in the epidermis, wherein the peripheral intra-epidermal nerve fiber is an unmyelinated fiber in the epidermis of the human skin,
and wherein the nonlinear optical microscopy device includes: a laser source for emitting laser light with a pulsed laser 320, and an image processing member 330 for processing image signals, in which the laser light is focused on the intra-epidermal nerve fiber of the acquisition area of the to-be-tested human skin by using the image processing member to obtain continuous signals of the intra-epidermal nerve fiber structural image with a length having at least three points and continuous, wherein the continuous signals of the intra-epidermal nerve fiber structural image are third harmonic generation nonlinear optical signals generated after being excited by the laser light, the size of the signal point of each of the continuous signals of the intra-epidermal nerve fiber structural image is 200 nm or more, and the linear distance from one signal point to another signal point in a three-dimensional space ranges from 0 to 7.5 µm, and wherein the continuous signals connected together by at least three points constitute a string-like intra-epidermal nerve fiber structural signal segment, and the string-like intra-epidermal nerve fiber structural signal segment includes signal segments only existing in the epidermis, and the other signal segments extendedly crossing through the junctional layer of the skin epidermis and dermis to exist in the epidermis, and the shortest distance between the string-like intra-epidermal nerve fiber structural signal segments and the junctional layer of the epidermis and dermis is less than 20µm, thereby constituting a plurality of nerve fibers;
a calculation element 340 for calculating a total number of the plurality of nerve fibers of the total intra-epidermal nerve fiber in the acquisition area of the to-be-tested human skin and dividing it by a total acquisition area (mm²) of the captured images to obtain a density of the intra-epidermal nerve fiber of the to-be-tested human skin; and
an evaluation and judging element 350 for evaluating levels of damage to the intra-epidermal nerve fiber of the to-be-tested human skin, and determining whether related neuropathy such as peripheral neuropathy is suffered, that is, the more severe the intra-epidermal nerve fiber is damaged, the lower the density is.

In the invention, the apparatus for non-invasive image-observing the density of an intra-epidermal nerve fiber of human skin 300 further comprises a displaying element 360 for displaying the image and/or the density of each nerve fiber.

In the invention, a normal value of the density of the intra-epidermal nerve fiber is from 50 to 60 (the number of the nerve fibers/mm²), and the intra-epidermal nerve fiber is a C nerve fiber or A-delta nerve fiber, having a point-like structure.

Further, in the apparatus for non-invasive image-observing the density of an intra-epidermal nerve fiber of human skin 300 of the invention, the non-linear optical microscopy device is a third harmonic generation microscopy device (THG), a second harmonic generation microscopy device (SHG) plus a third harmonic generation microscopy device (THG), or a combination thereof, wherein the specification of the third harmonic generation microscopy device (THG) is NA≧0.75, the central wavelength: 1065-1450nm, pulse width: < 10ps, and reverse collection of optical signals.

According to the apparatus for non-invasive image-observing the density of an intra-epidermal nerve fiber of human skin 300 of the invention, each nerve fiber extends along the same direction or a plurality of directions; or
each nerve fiber is continuous in the same epidermis with no extension, or is continuous in different skin layers with extensions; or when each nerve fiber extends in different skin layers, it is a point-like signal and a point-like signal extension, or a point-like signal and a linear signal extension; or each nerve fiber is not arranged as a circle to eliminate cell signals in the skin.

In addition, in the apparatus for non-invasive image-observing the density of an intra-epidermal nerve fiber of human skin 300 of the invention, the nonlinear optical microscopy device is a second harmonic generation microscopy device (SHG) plus a third harmonic generation microscopy device (THG) for observing the intra-epidermal nerve fiber structural image, and if the intra-epidermal nerve fiber extends in the horizontal direction (perpendicular to the forward direction of the laser light), and as is known through numerical simulation, it is used for generating a high-strength third harmonic generation, so that the intra-epidermal nerve fiber signal is observed in the same skin layer as linear and continuous; and if the intra-epidermal nerve fiber extends in the vertical direction (parallel to the forward direction of the laser light), it is known through numerical simulation, that if it is a simple cylindrical thin line, the intensity of the third harmonic generation is extremely small, it must conform to the structure of the varicosity to generate high-strength third harmonic generation, so that the intra-epidermal nerve fiber signal is observed in different skin layers as a point-like and point-like extension, or a point-like and linear extension.

Still further, the apparatus for non-invasive image-observing the density of an intra-epidermal nerve fiber of human skin 300 of the invention further comprises a correction element 370. When observing the intra-epidermal nerve fiber image, the number of cells in a basal layer of the epidermis capable of reflecting visibility of the image is further observed, and wherein a correction element is further provided for correcting the density of the intra-epidermal nerve fiber, via using the number of cells in the basal layer.

The test data of calculating the density of the intra-epidermal using the apparatus of the invention is presented in the above method as submitted.

STEPS IMPLEMENTED IN THE METHOD AND THE ELEMENTS EMPLOYED IN THE APPARATUS
- 100: A method for non-invasive image-observing the density of an intra-epidermal nerve fiber of human skin
- 110: providing a non-invasive, nonlinear optical microscopy device for capturing an intra-epidermal nerve fiber structural image of a to-be-tested human skin to observe continuous signals of intra-epidermal nerve fiber structural images
- 120: focusing the laser light, emitted by a laser source of the nonlinear optical microscopy device, on the intra-epidermal nerve fiber of the acquisition area of the to-be-tested human skin to obtain continuous signals of the intra-epidermal nerve fiber structural image having a length of at least three points, wherein the continuous connected together constitute a plurality of linear fibers
- 130: calculating a total number of the plurality of nerve fibers of the total intra-epidermal nerve fiber in the acquisition area of the to-be-tested human skin and dividing it by a total acquisition area (mm²) of the captured images to obtain a density of the intra-epidermal nerve fiber of the to-be-tested human skin; and
- 140: evaluating levels of damage to the intra-epidermal nerve fiber of the to-be-tested human skin, and determining whether related neuropathy, such as peripheral neuropathy is suffered, that is, the more severe the intra-epidermal nerve fiber is damaged, the lower the density is.
- 150: displaying the image and/or the density of each nerve fiber
- 170: using the number of cells in a basal layer of the epidermis simultaneously observed by using the nonlinear optical microscopy device to correct the density (value) of the entire segment of the intra-epidermal fiber and the density of the intra-epidermal nerve fiber
- 300: An apparatus for non-invasive image-observing the density of an intra-epidermal nerve fiber of human skin
- 310: a non-linear optical microscopy device
- 320: a laser source
- 330: an image processing member
- 340: a calculation element
- 350: evaluating and judging element
- 360: displaying element
- 370: correction element

## Claims

1. An apparatus for non-invasive image-observing the density of an intra-epidermal nerve fiber of human skin (300), which comprises:
a nonlinear optical microscopy device (310) for capturing an intra-epidermal nerve fiber structural image of an acquisition area of a to-be-tested human skin to observe continuous signals of the intra-epidermal nerve fiber structural image,
wherein the continuous signals of the intra-epidermal nerve fiber structural image include a string-like or a claw-like structural signal of a peripheral intra-epidermal nerve fiber formed by crossing through a junctional layer of the human skin epidermis and dermis and extending into the epidermis, and a string-like or a claw-like structural signal of the peripheral intra-epidermal nerve fiber only located in the epidermis,
wherein the peripheral intra-epidermal nerve fiber is an unmyelinated fiber in the epidermis of the human skin, and wherein the nonlinear optical microscopy device includes:
a laser source for emitting laser light with a pulsed laser (320), and
an image processing member (330) for processing image signals, in which the laser light is focused on the intra-epidermal nerve fiber of the acquisition area of the to-be-tested human skin by using the image processing member to obtain continuous signals of the intra-epidermal nerve fiber structural image with a length having at least three points and continuous,
wherein the continuous signals of the intra-epidermal nerve fiber structural image are third harmonic generation nonlinear optical signals generated due to excitation of the laser light, the size of the signal point of each of the continuous signals of the intra-epidermal nerve fiber structural image is 200 nm or more, and the linear distance from one signal point to another signal point in a three-dimensional space ranges from 0 to 7.5 µm, and
wherein the continuous signals connected together by at least three points constitute one string-like intra-epidermal nerve fiber structural signal segment, and the one string-like intra-epidermal nerve fiber structural signal segment includes signal segments only existing in the epidermis, and other signal segments extendedly crossing through the junctional layer of the skin epidermis and dermis to exist in the epidermis, and the shortest distance between the one string-like intra-epidermal nerve fiber structural signal segment and the junctional layer of the epidermis and dermis is less than 20µm, thereby constituting one nerve fiber of a plurality of nerve fibers observed for calculation;
a calculation element (340) for calculating a total number of the plurality of nerve fibers of the total intra-epidermal nerve fiber in the acquisition area of the to-be-tested human skin and dividing it by a total acquisition area (mm²) of the captured images to obtain a density of the intra-epidermal nerve fiber of the to-be-tested human skin; and
an evaluation and judging element (350) for evaluating levels of damage to the intra-epidermal nerve fiber of the to-be-tested human skin, and determining whether related neuropathy such as peripheral neuropathy is suffered, that is, the more severe the intra-epidermal nerve fiber is damaged, the lower the density is.

2. The apparatus for non-invasive image-observing the density of an intra-epidermal nerve fiber of human skin (300) of claim 1, which further comprises a displaying element (360) for displaying the image and/or the density of each nerve fiber.

3. The apparatus for non-invasive image-observing the density of an intra-epidermal nerve fiber of human skin (300) of claim 1 or 2, wherein a normal value of the density of the intra-epidermal nerve fiber is from 50 to 60 (the number of the nerve fibers/mm2).

4. The apparatus for non-invasive image-observing the density of an intra-epidermal nerve fiber of human skin (300) of claim 1, 2 or 3, wherein the intraepidermal nerve fiber is a C nerve fiber or A-delta nerve fiber, having a point-like structure.

5. The apparatus for non-invasive image-observing the density of an intra-epidermal nerve fiber of human skin (300) of any one of the preceding claims 1-4, wherein the non-linear optical microscopy device is a third harmonic generation microscopy device (THG), a second harmonic generation microscopy device (SHG) plus a third harmonic generation microscopy device (THG), or a combination thereof, wherein the specification of the third harmonic generation microscopy device (THG) is NA≧0.75, the central wavelength: 1065-1450nm, pulse width: < 10ps, and reverse collection of optical signals.

6. The apparatus for non-invasive image-observing the density of an intra-epidermal nerve fiber of human skin (300) of any one of the preceding claims 1-5, wherein each nerve fiber extends along the same direction or a plurality of directions.

7. The apparatus for non-invasive image-observing the density of an intra-epidermal nerve fiber of human skin (300) of any one of the preceding claims 1-6, wherein each nerve fiber is continuous in the same epidermis with no extension, or is continuous in different skin layers with extensions.

8. The apparatus for non-invasive image-observing the density of an intra-epidermal nerve fiber of human skin (300) of any one of the preceding claims 1-7, wherein when each nerve fiber extends in different skin layers, it is a point-like signal and a point-like signal extension, or a point-like signal and a linear signal extension.

9. The apparatus for non-invasive image-observing the density of an intra-epidermal nerve fiber of human skin (300) of any one of the preceding claims 1-8, wherein each nerve fiber is not arranged as a circle to eliminate cell signals in the skin.

## Patentansprüche

1. Vorrichtung für eine nichtinvasive Bildbeobachtung der Dichte einer intraepidermalen Nervenfaser menschlicher Haut (300), die umfasst:
eine nichtlineare optische Mikroskopvorrichtung (310) zum Aufnehmen eines Strukturbildes einer intraepidermalen Nervenfaser eines Erfassungsbereichs einer zu testenden menschlichen Haut, um kontinuierliche Signale des Strukturbildes einer intraepidermalen Nervenfaser zu beobachten,
wobei die kontinuierlichen Signale des Strukturbildes einer intraepidermalen Nervenfaser ein strangähnliches oder klauenähnliches strukturelles Signal einer peripheren intraepidermalen Nervenfaser, die durch Durchqueren einer Verbindungsschicht der Epidermis und der Dermis der menschlichen Haut und Erstrecken in die Epidermis gebildet wird, und ein strangähnliches oder klauenähnliches strukturelles Signal einer peripheren intraepidermalen Nervenfaser, die sich nur in der Epidermis befindet, enthalten,
wobei die periphere intraepidermale Nervenfaser eine markscheidenlose Faser in der Epidermis der menschlichen Haut ist und wobei die nichtlineare optische Mikroskopvorrichtung enthält:
eine Laserquelle zum Aussenden von Laserlicht mit einem gepulsten Laser (320) und
ein Bildverarbeitungselement (330) zum Verarbeiten von Bildsignalen, in denen das Laserlicht auf eine intraepidermale Nervenfaser des Erfassungsbereichs der zu testenden menschlichen Haut durch Verwenden des Bildverarbeitungselements gebündelt ist, um kontinuierliche Signale des Strukturbildes einer intraepidermalen Nervenfaser mit einer Länge, die mindestens drei Punkte besitzt und durchgängig ist, zu erhalten,
wobei die kontinuierlichen Signale des Strukturbildes einer intraepidermalen Nervenfaser nichtlineare optische Signale einer dritten harmonischen Erzeugung sind, die aufgrund der Anregung des Laserlichts erzeugt werden, wobei die Größe des Signalpunkts jedes der kontinuierlichen Signale des Strukturbildes einer intraepidermalen Nervenfaser 200 nm oder mehr beträgt und der lineare Abstand von einem Signalpunkt zu einem anderen Signalpunkt in einem dreidimensionalen Raum in einem Bereich von 0 bis 7,5 µm liegt, und
wobei die kontinuierlichen Signale, die miteinander durch mindestens drei Punkte verbunden sind, ein strangähnliches strukturelles Signalsegment einer intraepidermalen Nervenfaser bilden und das strukturelle Signalsegment einer intraepidermalen Nervenfaser Signalsegmente, die nur in der Epidermis vorhanden sind, und andere Signalsegmente, die erweitert die Verbindungsschicht der Hautepidermis und -dermis durchqueren, um in der Epidermis vorhanden zu sein, enthält und der kürzeste Abstand zwischen dem einen strangähnlichen strukturellen Signalsegment einer intraepidermalen Nervenfaser und der Verbindungsschicht der Epidermis und Dermis weniger als 20 µm beträgt, wodurch eine Nervenfaser mehrerer Nervenfasern, die für eine Berechnung beobachtet werden, gebildet wird;
ein Berechnungselement (340) zum Berechnen einer Gesamtanzahl der mehreren Nervenfasern der gesamten intraepidermalen Nervenfaser in dem Erfassungsbereich der zu testenden menschlichen Haut und Dividieren derselben durch einen Gesamterfassungsbereich (mm²) der aufgenommenen Bilder, um eine Dichte der intraepidermalen Nervenfaser der zu testenden menschlichen Haut zu erhalten; und
ein Einschätzungs- und Beurteilungselement (350) zum Einschätzen von Schadensniveaus der intraepidermalen Nervenfaser der zu testenden menschlichen Haut und Bestimmen, ob eine zugeordnete Neuropathie, wie etwa eine periphere Neuropathie vorliegt, das heißt, je schwerer die einer intraepidermalen Nervenfaser beschädigt ist, desto geringer ist die Dichte.

2. Vorrichtung zur nichtinvasiven Bildbeobachtung der Dichte einer intraepidermalen Nervenfaser menschlicher Haut (300) nach Anspruch 1, die ferner ein Anzeigeelement (360) zum Anzeigen des Bildes und/oder der Dichte jeder Nervenfaser umfasst.

3. Vorrichtung zur nichtinvasiven Bildbeobachtung der Dichte einer intraepidermalen Nervenfaser menschlicher Haut (300) nach Anspruch 1 oder 2, wobei ein normaler Wert der Dichte zwischen 50 und 60 (die Anzahl der Nervenfasern/mm2) liegt.

4. Vorrichtung zur nichtinvasiven Bildbeobachtung der Dichte einer intraepidermalen Nervenfaser menschlicher Haut (300) nach Anspruch 1, 2 oder 3, wobei die intraepidermale Nervenfaser eine C-Nervenfaser oder eine A-Delta-Nervenfaser mit einer punktähnlichen Struktur ist.

5. Vorrichtung zur nichtinvasiven Bildbeobachtung der Dichte einer intraepidermalen Nervenfaser menschlicher Haut (300) nach einem der vorhergehenden Ansprüche 1-4, wobei die nichtlineare optische Mikroskopvorrichtung eine Mikroskopvorrichtung einer dritten harmonischen Erzeugung (THG), eine Mikroskopvorrichtung einer zweiten harmonischen Erzeugung (SHG) plus eine Mikroskopvorrichtung eine dritten harmonischen Erzeugung (THG) oder eine Kombination davon ist, wobei die Spezifikation der Mikroskopvorrichtung einer dritten harmonischen Erzeugung (THG) NA ≥ 0,75 ist, die zentrale Wellenlänge: 1065-1450 nm ist, die Impulsbreite: <10 ps ist und eine umgekehrte Sammlung optischer Signale.

6. Vorrichtung zur nichtinvasiven Bildbeobachtung der Dichte einer intraepidermalen Nervenfaser menschlicher Haut (300) nach einem der vorhergehenden Ansprüche 1-5, wobei sich jede Nervenfaser entlang derselben Richtung oder mehrerer Richtungen erstreckt.

7. Vorrichtung zur nichtinvasiven Bildbeobachtung der Dichte einer intraepidermalen Nervenfaser menschlicher Haut (300) nach einem der vorhergehenden Ansprüche 1-6, wobei jede Nervenfaser in derselben Epidermis ohne Erweiterung durchgängig ist oder in verschiedenen Hautschichten mit Erweiterungen durchgängig ist.

8. Vorrichtung zur nichtinvasiven Bildbeobachtung der Dichte einer intraepidermalen Nervenfaser menschlicher Haut (300) nach einem der vorhergehenden Ansprüche 1-7, wobei dann, wenn sich jede Nervenfaser in verschiedenen Hautschichten erstreckt, es ein punktähnliches Signal und eine punktähnliche Signalerweiterung oder ein punktähnliches Signal und eine lineare Signalerweiterung ist.

9. Vorrichtung zur nichtinvasiven Bildbeobachtung der Dichte einer intraepidermalen Nervenfaser menschlicher Haut (300) nach einem der vorhergehenden Ansprüche 1-8, wobei jede Nervenfaser nicht als ein Kreis angeordnet ist, um Zellsignale in der Haut zu beseitigen.

## Revendications

1. Appareil pour l'observation par imagerie non invasive de la densité d'une fibre nerveuse intra-épidermique d'une peau humaine (300), qui comporte :
un dispositif de microscopie optique non linéaire (310) pour capturer une image structurelle de fibre nerveuse intra-épidermique d'une zone d'acquisition d'une peau humaine à tester pour observer des signaux continus de l'image structurelle de fibre nerveuse intra-épidermique,
dans lequel les signaux continus de l'image structurelle de fibre nerveuse intra-épidermique incluent un signal structurel analogue à une chaîne ou analogue à une griffe d'une fibre nerveuse intra-épidermique périphérique formée par croisement à travers une couche de jonction de l'épiderme et du derme de peau humaine et s'étendant dans l'épiderme, et un signal structurel analogue à une chaîne ou analogue à une griffe de la fibre nerveuse intra-épidermique périphérique uniquement située dans l'épiderme,
dans lequel la fibre nerveuse intra-épidermique périphérique est une fibre amyélinique dans l'épiderme de la peau humaine, et dans lequel le dispositif de microscopie optique non linéaire inclut :
une source laser pour émettre de la lumière laser avec un laser pulsé (320), et
un élément de traitement d'image (330) pour traiter des signaux d'image, dans lequel la lumière laser est focalisée sur la fibre nerveuse intra-épidermique de la zone d'acquisition de la peau humaine à tester en utilisant l'élément de traitement d'image pour obtenir des signaux continus de l'image structurelle de fibre nerveuse intra-épidermique ayant une longueur d'au moins trois points et continue,
dans lequel les signaux continus de l'image structurelle de fibre nerveuse intra-épidermique sont des signaux optiques non linéaires de génération d'harmoniques de rang 3 générés en raison d'une excitation de la lumière laser, la taille du point de signal de chacun des signaux continus de l'image structurelle de fibre nerveuse intra-épidermique est de 200 nm ou plus, et la distance linéaire d'un point de signal à un autre point de signal dans l'espace tridimensionnel varie de 0 à 7,5 µm, et
dans lequel les signaux continus reliés ensemble par au moins trois points constituent un segment de signal structurel de fibre nerveuse intra-épidermique analogue à une chaîne, et le segment de signal structurel de fibre nerveuse intra-épidermique analogue à une chaîne inclut des segments de signal existant uniquement dans l'épiderme, et d'autres segments de signal se croisant de manière étendue à travers la couche de jonction de l'épiderme et du derme de peau pour exister dans l'épiderme, et la plus courte distance entre le segment de signal structurel de fibre nerveuse intra-épidermique analogue à une chaîne et la couche de jonction de l'épiderme et du derme est inférieure à 20 µm, en constituant ainsi une fibre nerveuse parmi une pluralité de fibres nerveuses observées en vue d'un calcul ;
un élément de calcul (340) pour calculer un nombre total de la pluralité de fibres nerveuses de la fibre nerveuse intra-épidermique totale dans la zone d'acquisition de la peau humaine à tester et le diviser par une surface d'acquisition totale (mm²) des images capturées afin d'obtenir une densité de la fibre nerveuse intra-épidermique de la peau humaine à tester ; et
un élément d'évaluation et de jugement (350) pour évaluer des niveaux d'endommagement de la fibre nerveuse intra-épidermique de la peau humaine à tester, et déterminer si une neuropathie associée telle qu'une neuropathie périphérique est subie, c'est-à-dire que plus la fibre nerveuse intra-épidermique est endommagée sévèrement, plus la densité est faible.

2. Appareil pour l'observation par imagerie non invasive de la densité d'une fibre nerveuse intra-épidermique d'une peau humaine (300) selon la revendication 1, qui comporte en outre un élément d'affichage (360) pour afficher l'image et/ou la densité de chaque fibre nerveuse.

3. Appareil pour l'observation par imagerie non invasive de la densité d'une fibre nerveuse intra-épidermique d'une peau humaine (300) selon la revendication 1 ou 2, dans lequel une valeur normale de la densité de la fibre nerveuse intra-épidermique est de 50 à 60 (le nombre des fibres nerveuses/mm2).

4. Appareil pour l'observation par imagerie non invasive de la densité d'une fibre nerveuse intra-épidermique d'une peau humaine (300) selon la revendication 1, 2 ou 3, dans lequel la fibre nerveuse intra-épidermique est une fibre nerveuse C ou une fibre nerveuse A delta, ayant une structure ponctiforme.

5. Appareil pour l'observation par imagerie non invasive de la densité d'une fibre nerveuse intra-épidermique d'une peau humaine (300) selon l'une quelconque des revendications 1 à 4 précédentes, dans lequel le dispositif de microscopie optique non linéaire est un dispositif de microscopie de génération d'harmoniques de rang 3 (THG), un dispositif de microscopie de génération d'harmoniques der rang 2 (SHG) plus un dispositif de microscopie de génération d'harmoniques de rang 3 (THG), ou une combinaison de ceux-ci, dans lequel la spécification du dispositif de microscopie de génération d'harmoniques de rang 3 (THG) est NA ≥ 0,75, la longueur d'onde centrale : 1065-1450 nm, largeur d'impulsion : < 10 ps, et collecte inversée de signaux optiques.

6. Appareil pour l'observation par imagerie non invasive de la densité d'une fibre nerveuse intra-épidermique d'une peau humaine (300) selon l'une quelconque des revendications 1 à 5 précédentes, dans lequel chaque fibre nerveuse s'étend le long de la même direction ou d'une pluralité de directions.

7. Appareil pour l'observation par imagerie non invasive de la densité d'une fibre nerveuse intra-épidermique d'une peau humaine (300) selon l'une quelconque des revendications 1 à 6 précédentes, dans lequel chaque fibre nerveuse est continue dans le même épiderme sans extension, ou est continue dans différentes couches de la peau avec des extensions.

8. Appareil pour l'observation par imagerie non invasive de la densité d'une fibre nerveuse intra-épidermique d'une peau humaine (300) selon l'une quelconque des revendications 1 à 7 précédentes, dans lequel lorsque chaque fibre nerveuse s'étend dans différentes couches de la peau, il s'agit d'un signal ponctiforme et d'une extension de signal ponctiforme, ou d'un signal ponctiforme et d'une extension de signal linéaire.

9. Appareil pour l'observation par imagerie non invasive de la densité d'une fibre nerveuse intra-épidermique d'une peau humaine (300) selon l'une quelconque des revendications 1 à 8 précédentes, dans lequel chaque fibre nerveuse n'est pas disposée en cercle pour éliminer des signaux de cellules dans la peau.
